# EUROPEAN PATENT APPLICATION

(11) **EP 3 991 737 A1**
(43) Date of publication of application: **04.05.2022**
(21) Application number: 20832054.9
(22) Date of filing: 15.06.2020
(51) Int. Cl.: A61K 33/04, A61M 15/00, A61M 35/00, A61M 37/00, A61P 17/02

(54) **GAS COMPOSITION FOR WOUND TREATMENT USE, AND WOUND TREATMENT DEVICE**

(30) Priority: 28.06.2019 JP 2019122423
(71) Applicant: Sumitomo Seika Chemicals Co., Ltd., Kako-gun, Hyogo 675-0145 (JP); University of The Ryukyus, Nakagami-gun, Okinawa 903-0213 (JP)
(72) Inventor: SAKAUE Shigeki, Kako-gun, Hyogo 675-0145 (JP); KAKINOHANA Manabu, Nakagami-gun, Okinawa 903-0213 (JP)
(74) Representative: Vossius & Partner Patentanwälte Rechtsanwälte mbB
(86) International application number: PCT/JP2020/023342
(87) International publication number: WO 2020/262055

(57) **Abstract**

The invention provides a wound-treating gas composition and a wound-treating apparatus usable with a novel wound-treating method making use of hydrogen sulfide. The wound-treating gas composition contains hydrogen sulfide in a proportion of 20 ppm or lower, and the wound-treating apparatus comprises a cover or container to which the wound-treating gas is fed.

## Description

### Field of the Art

The present invention relates generally to a gas composition and apparatus configured to treat wounds.

### Background Art

Being physical damage to surface tissues of the body, the wound is a general term for wounds with or without openings.

In most cases, a slight wound heals over naturally without recourse to any particular treatment. A severe wound usually heals over by way of natural healing power, too, if it is properly treated by suturing, skin grafting, etc.

In no little cases, however, wound healing takes too long time with respect to intractable ulcers such as bedsores, venous stasis ulcers, arterial ulcers, diabetic ulcers and radiation ulcers. In some cases, it takes too long time for patients undergoing an operation to heal operative wound primarily because of a lowering of their natural healing power caused by their own diseases or medication.

Of such intractable wounds, the bedsore observed in long bedridden patients is a necrotic skin ulcer caused by a long-standing application of certain or higher pressures to the skin.

In recent years, with aging of population, patients suffering from bedsores while spending long confined to bed have tended to increase in number. To deal with this situation, various proposals of therapeutic drugs or methods for treating wounds inclusive of bedsores.

For instance, Patent Publication 1 discloses that sodium glutamate is orally administrated to patients in the form of an internal medicine, and Patent Publication 2 discloses that a wound treating agent containing glutamine, polydexstrose, lactulose and befidobacteria is orally administrated to patients.

Referring to an external preparation and its administration method, Patent Publication 3 discloses that an external preparation containing either one of hydrophilic or white petrolatum and povidone iodine is coated on a wound surface. Also, Patent Publication 4 discloses that hydrogen-containing water is used as a liquid for external use in which a bedsore site is immersed, hydrogen-containing water is added dropwise onto the bedsore site and a coating means soaked with the hydrogen-containing water is affixed to the bedsore site. Further, Patent Publication 5 discloses that cotton gauze soaked with fradiomycin sulfate and trafermin is packed in the back of an ulcer. Furthermore, Patent Publication 6 discloses that a wound coating material composed of an unwoven fabric containing crosslinked hyaluronic acid, silver sulfadiazine and crosslinked alginic acid is applied to a wound surface.

On the other hand, hydrogen sulfide (H₂S)-containing spa has been used for folk remedies from old, because it has been known to be efficacious against skin diseases or circulatory diseases. However, hydrogen sulfide (H₂S) is difficult to handle on a daily basis thanks to its toxicity. In recent years, this hydrogen sulfide has been reported to have bioactivities such as cell protective action, blood vessel relaxing action, antioxidant action, neurotransmission regulation action and apoptosis inhibition action under low concentration conditions.

As such biological activities of hydrogen sulfide have been clearly understood, brisk trials of applying this to medical treatments are now under way.

For instance, Patent Publication 7 discloses that viablilty of mice is improved when they are exposed to a H₂S atmosphere at 80 ppm to place them in "stasis conditions" where their activity is recovered.

Patent Publication 8 discloses a technical idea wherein a therapeutic gas having vasodilatory action such as hydrogen sulfide is stored in a biocompatible polymer matrix containing a polymer and closed cells and comprising a surface configured to come in direct contact with a wound site thereby delivering said therapeutic gas to said wound site.

### Prior Arts

### Patent Publications

Patent Publication 1: JP(A) 2009-191015
Patent Publication 2: JP(A) 2015-120646
Patent Publication 3: JP(A) 2010-77143
Patent Publication 4: JP(A) 2011-219411
Patent Publication 5: JP(A) 2016-77514
Patent Publication 6: JP(A) 2001-212170
Patent Publication 7: Japanese Unexamined Patent Application Publication No. 2008-538569
Patent Publication 8: Japanese Unexamined Patent Application Publication No. 2019-507622

### Summary of the Invention

### Problems to be Solved by the Invention

As described above, various methods have so far been reported to treat wounds inclusive of bedsores, and various medical treatment methods harnessing the bioactivity of hydrogen sulfide are reported too. However, there is no report so far about curing a wound by exposing it to hydrogen sulfide or drawing hydrogen sulfide into lungs.

Accordingly, the present invention has for its object to provide a wound-treating gas composition and apparatus that is used with a novel wound-treating method making use of hydrogen sulfide.

### Embodiment of the Invention

As a result of various studies made for the purpose of achieving the aforesaid object, the present inventors have accomplished the present invention by finding that healing effect or efficacy of a wound is improved as the wound is exposed to a gas composition containing a constant amount of hydrogen sulfide or this gas composition is allowed to be inhaled in the living organism to be treated.

The first embodiment of the invention configured to achieve the aforesaid object provides a wound-treating gas composition containing hydrogen sulfide in a proportion of 20 ppm or lower.

The second embodiment of the invention configured to achieve the aforesaid object provides a wound-treating apparatus comprising a cover or container to receive the aforesaid wound-treating gas composition to expose a wound to the aforesaid gas composition and/or allow the living organism to be treated to inhale the aforesaid gas composition.

### Advantages of the Invention

According to the invention disclosed herein, it is possible to provide a wound-treating gas composition and apparatus usable with a novel wound-treating method making use of hydrogen sulfide.

### Brief Explanation of the Drawings

Fig. 1 is a conceptual view illustrating the construction of the wound-treating apparatus according to one embodiment of the invention.
Fig. 2 is illustrative of bedsore formation employed in Examples 1 to 4 and Comparative Examples 1 and 2, respectively.
Fig. 3 is illustrative of the protocol for hydrogen sulfide-containing gas delivery or feed and bedsore area measurement used in Examples 1 to 4 and Comparative Examples 1 and 2, respectively.
Fig. 4 is a set of photographs showing bedsore states in Examples 1 to 4 and Comparative Examples 1 and 2, respectively ((a): Comp. Ex. 1. (b): Comp. Ex. 2, (c): Ex. 1, (d): Ex. 2, (e): Ex. 3, (f): Ex. 4).
Fig. 5 is a set of graphs showing the results of bedsore area measurements in Examples 1 to 4 and Comparative Example 2, respectively, in comparison with the results of Comparative Example 1 ((a): Comp. Ex. 2, (b): Ex. 1, (c): Ex. 2, (d): Ex. 3, (e): Ex. 4).
Fig. 6 is illustrative of the protocol for hydrogen sulfide-containing gas delivery or feed and skin excision area measurement used in Example 5 and Comparative Example 3, respectively.
Fig. 7 is a set of photographs showing skin excision states in Examples 5 to 7 and Comparative Example 3, respectively ((a): Ex. 5, (b): Ex. 6, (c): Ex. 7, (d): Comp. Ex. 3).
Fig. 8 is a set of graphs showing the results of skin excision area measurement in Examples 5 to 7, respectively, in comparison with the results of Comparative Example 3 ((a): Ex. 5, (b): Ex. 6, (c): Ex. 7) .
Fig. 9 is a set of photographs showing skin excision states in Example 8 and Comparative Example 4 ((a): Ex. 8, (b): Comp. Ex. 4).
Fig. 10 is a graph showing the results of skin excision area measurement in Example 8 and Comparative Example 4.

### Modes for Carrying Out the Invention

By way of example but not by way of limitation, the present invention is now explained in greater details with reference to one embodiment thereof.

### Gas Composition

The wound-treating gas composition according to the first embodiment of the invention (hereinafter called the "first embodiment") contains hydrogen sulfide in a proportion of 20 ppm or lower.

The concentration of hydrogen sulfide in the gas composition to ensure that such a high therapeutic efficacy is obtainable is 20 ppm or lower. As the concentration of hydrogen sulfide is greater than 20 ppm, it gives rise to a decrease in therapeutic efficacy against wounds. Although the cause of this decrease has yet to be clarified, it would be related to the toxicity of hydrogen sulfide. The upper limit value to the concentration of hydrogen sulfide in the gas composition is preferably 10 ppm or lower at which there is wound healing efficacy obtained earlier, more preferably 5 ppm or lower, and most preferably 1 ppm or lower.

There is no lower limit value to the concentration of hydrogen sulfide as long as the gas composition contains hydrogen sulfide, but it is preferably at least 0.4 ppb, and more preferably at least 0.02 ppm in terms of healing acceleration.

It is here understood that the hydrogen sulfide concentration is given on a volume basis.

The gas composition according to the first embodiment is noticeably efficacious against wounds, in particular wounds difficult to heal over naturally, for instance, bedsores.

### [Wound-Treating Apparatus]

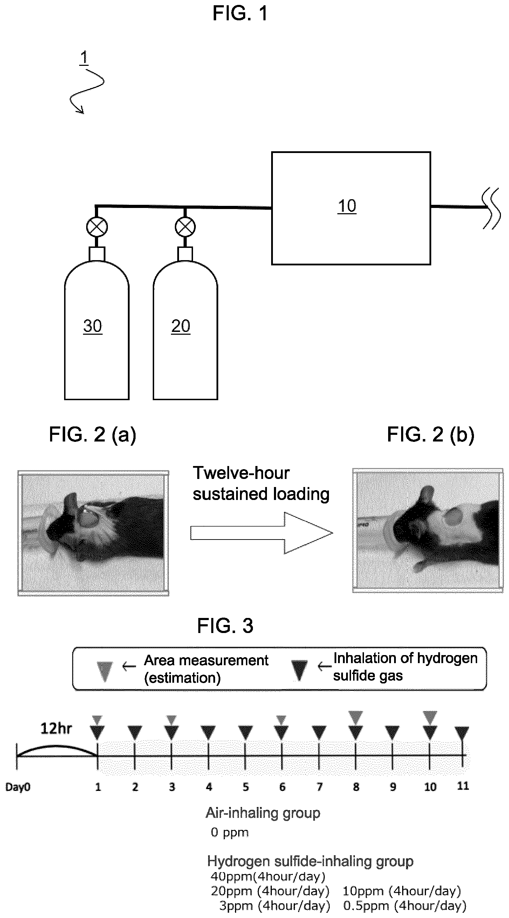

As shown in Fig. 1, a wound-treating apparatus 1 according to the second embodiment of the invention (hereafter called the "second embodiment") comprises a cover or container 10 configured to receive the wound-treating gas composition containing hydrogen sulfide in a proportion of 20 ppm or lower so that a wound can be exposed to said gas composition and/or the living organism to be treated is allowed to inhale said gas composition.

Although the mechanism through which the aforesaid gas composition affects the treatment of wounds has yet to be clarified, it would be related to skin tissue changes by contact with the gas composition and improvements in the natural healing power caused by inhalation of the aforesaid gas composition. Therefore, it would be possible to boost up the healing of the wound noticeably if the aforesaid gas composition is fed to the cover or container 10 and a wound is exposed to hydrogen sulfide contained in the aforesaid gas composition and/or the living organism to be treated is allowed to inhale said hydrogen sulfide by the aforesaid apparatus construction.

The cover or container 10 used herein has a function of exposing a wound to the wound-treating gas composition, and a function of allowing the living organism to be treated to inhale said gas composition. The cover used herein is understood to refer to a member configured to cover a wound or the mouth or nose of the living organism to be treated, as exemplified by a shell-structured or sheet-shaped member. The container used herein is understood to stand for a member configured to take in a wound site or the mouth, nose or whole body of the living organism to be treated inside, as exemplified by a box-shaped member. There is no limitation on the material, structure, etc. of the cover or container 10 provided that they are incapable of reacting with hydrogen sulfide, and prevent a release of the hydrogen sulfide-containing gas around the apparatus. One example of the cover material includes hydrogenated styrene-base thermoplastic elastomer (SEBS), thermoplastic elastomer (TPE) or the like, and one example of the container material includes acrylic resin, glass or the like. The cover or container 10 may be made up of a plurality of materials. One example of the structure includes the one having an inlet and an outlet for the hydrogen sulfide-containing gas, the one constituted by a combination of plural members assembled by way of a gasket or the like, or the one having a door portion which the living organism to be treated goes in and out, and so on. The cover or container 10 is preferably a closable container in which the whole body of the living organism to be treated is taken or enclosed because it is easy to prevent the hydrogen sulfide-containing gas from leaking to the outside during use, and exposure to the delivered wound-treating gas composition and inhalation of said gas composition take place at the same time. In that case, it is more preferable to form the cover or container of a transparent material in such a way as to check the condition of the living organism during treatment.

The wound-treating gas composition fed into the cover or container 10 should contain hydrogen sulfide in a proportion of 20 ppm or lower, as mentioned above. Feeding of said wound-treating gas composition contributes to such noticeable wound healing efficacies as mentioned above.

The wound-treating apparatus 1 according to the present embodiment comprises a wound-treating gas source 20 to feed the aforesaid wound-treating gas composition to the cover or container 10. There is no limitation on the wound-treating gas source 20 with the proviso that it is capable of feeding the wound-treating gas containing no or little impurities in a stable way. A bomb loaded or filled with the wound-treating gas or a compound that reacts with components in the air to generate hydrogen sulfide may be referred to as an example.

The wound-treating apparatus 1 according to the present embodiment may further comprise a carrier gas source 30 configured to dilute the hydrogen sulfide-containing gas fed out of the wound-treating gas source 20 and boost up its delivery to the cover or container 10. There is no limitation on the carrier gas source 30 with the proviso that it is capable of stable carrier gas delivery. For instance, a bomb loaded with the carrier gas may be mentioned.

There is also no limitation on the type of carrier gas used herein with the proviso that it remains stable in the air and does not interfere with the wound healing action of hydrogen sulfide. As an example, air, nitrogen, argon and mixture thereof may be mentioned, among which air is preferred because it is easily available and because even when the whole body of the living organism to be treated is enclosed in the container 10, breathing is well ensured.

The wound-treating apparatus 1 according to the present embodiment is preferably configured such that gas delivery and interruption take place intermittently within a preset time. In one exemplary configuration, a flow rate control means such as a needle valve and a valve controller or mass flow controller may be provided in a pathway from the wound-treating gas source 20 to the cover or container 10. When the wound-treating apparatus 1 is provided with the carrier gas source 30 as described above, the aforesaid flow rate control means is also preferably located in the pathway from said carrier gas source 30 to the cover or container 10.

Other embodiment associated in technical idea with the present invention (hereafter called the "associated embodiment") relates to an alternative wound-treating method comprising exposing a wound to the wound-treating gas composition according to the first embodiment and/or allowing the living organism to be treated to inhale said gas composition. According to said treating method, noticeable therapeutic effects on wounds are achievable by way of the action of the aforesaid wound-treating gas.

In the associated embodiment, there is no limitation on the means for exposing wounds to the wound-treating gas composition and/or allowing the living organism to be treated to inhale said gas composition; however, it is preferable to use the wound-treating apparatus according to the aforesaid second embodiment because the gas having a low hydrogen sulfide concentration can be fed in a stable manner.

### Examples

The present invention will now be specifically explained on the basis of examples; however, these examples are given as an aid for an easy understanding of the present invention, but not by way of limitation.

### [Example 1]

In Example 1 as well as Examples 2 to 4 and Comparative Examples 1 and 2 to be given later, the efficacy of the invention against a bedsore model was studied.

### <Provision of the living organism to be treated>

A mouse (C57BL/6 mouse at 8 weeks old) was anesthetized by isoflurane, and its back skin was put and pressed between two strong magnets as shown in Fig. 2(a) and held for 12 hours to create such a bedsore as depicted in Fig. 2(b).

### <Exposure to the hydrogen sulfide-containing gas and/or inhalation of said gas as well as observation of bedsores>

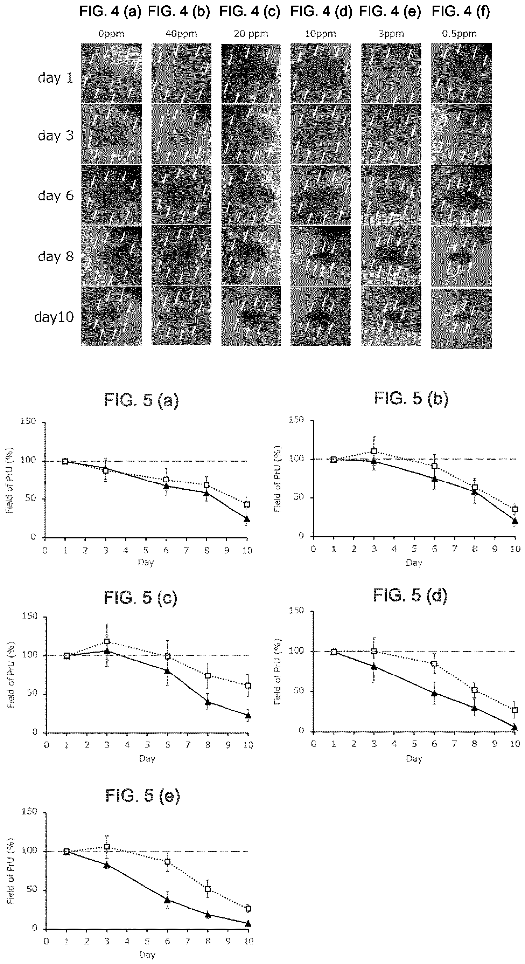

The aforesaid mouse was placed in the container 10 of the wound-treating apparatus 1 constructed as shown in Fig. 1. It is here noted that a box made from acrylic resin which is capable of receiving the whole body of the mouse and being closed up was used as the container 10. Subsequently, a gas having a hydrogen sulfide concentration of 20 ppm with air as a carrier gas was fed and the bedsore was observed according to the protocol shown in Fig. 3. Specifically, after an elapse of 12 hours from placement of the mouse in the container 10, the hydrogen sulfide-containing gas was fed to the container for 4 hours per day over 11 days. After an elapse of 1 day, 3 days, 6 days, 8 days, and 10 days from placement of the mouse in the container 10, photographs of the bedsore were taken under anesthesia by isoflurane, and submitted to a third person dermatologist to get him or her to use Image J (Fuji) to measure the number of pixels with a ruler width of 1 cm. Then, with 10 units as one unit, the bedsore site was surrounded and this unit was used for area calculation to make a morphological estimation of healing process with the first day as 100%. Photographs taken of the bedsore are attached hereto as Fig. 4(c), and the results of bedsore area measurement are shown by black triangular markers and a solid line in Fig. 5(b). Note here that the "Field of PrU (Pressure Ulcer)" in Fig. 5 stands for a bedsore area.

### [Examples 2 to 4]

The treatment according to Example 2, 3, and 4 was carried out as in Example 1 except that the concentrations of hydrogen sulfide in the gas fed into the container 10 were 10 ppm (Ex. 2), 3 ppm (Ex. 3), and 0.5 ppm (Ex. 4), respectively.

Referring to Example 2, photographs taken of the bedsore are attached hereto as Fig. 4(d), and the results of bedsore area measurement are indicated by black triangular markers and a solid line in 5€, respectively. Referring to Example 3, photographs taken of the bedsore are attached hereto as Fig. 4(e), and the results of bedsore area measurement are indicated by black triangular markers and a solid line in Fig. 5(d), respectively. Referring further to Example 4, photographs taken of the bedsore are attached hereto as Fig. 4(f), and the results of bedsore area measurement are indicated by black triangular markers and a solid line in Fig. 5(e), respectively.

### [Comparative Example 1]

The treatment according to Comparative Example 1 was carried out as in Example 1 except that the gas fed into the container 10 was only an air, or a gas having a hydrogen sulfide concentration of 0 ppm.

Photographs taken of the bedsore are attached hereto as Fig. 4(a), and the results of bedsore area measurement obtained herein are indicated by white square markers and dotted lines in Figs. 5(a) to 5(e), respectively.

### [Comparative Example 2]

The treatment according to Comparative Example 2 was carried out as in Example 1 except that the gas fed into the container 10 had a hydrogen sulfide concentration of 40 ppm.

Referring to Comparative Example 2, photographs taken of the bedsore are attached hereto as Fig. 4(b), and the results of bedsore area measurement are indicated by black circular markers and a solid line in Fig. 5(a), respectively.

From comparisons of Figs. 4(b) to 4(f) with Fig. 4(a), it has been identified that in the examples using hydrogen sulfide in a concentration of 20 ppm or lower as shown in Figs. 4(c) to 4(f), the bedsore state is noticeably improved on the tenth day at the latest. As shown in Figs. 4(d) to Fig. 4(f) in particular, it has been identified that in Examples 2 to 4 using a gas containing hydrogen sulfide of 10 ppm or lower, there are improvements in the bedsore state observed at the eighth day. As shown in Fig. 4(b), on the other hand, it has been identified that in Comparative Example 2 using 40 ppm hydrogen sulfide, no difference is found in the bedsore state between Figs. 4(a) and 4(b).

Referring to Fig. 5, it has been identified that the bedsore area becomes noticeably smaller as compared with the feeding of air alone, as shown in Figs. 5(c) to 5(e).

The aforesaid results would imply that exposing bedsores to a gas containing hydrogen sulfide in a proportion of 20 ppm or lower and/or allowing the living organism to inhale said gas composition induce healing efficacy against bedsores. Especially when the concentration of hydrogen sulfide in the gas is set at 10 ppm or lower, noticeable healing efficacies would be obtained.

### [Example 5]

In Example 5 as well as Examples 6 and 7 and Comparative Example 3 to be given later, the effect of the invention on a skin excision model was examined.

### <Provision of the living organism to be treated>

One day after a mouse (C57BL/6 mouse at 8-weeks old) was preoperatively shaven off at its back, it was anesthetized by isoflurane to hollow out the back skin through the cuticle and the derma using a biopsy trepan (made by Kai Industries, Co., Ltd.) having a diameter of 6 mm.

### <Exposure to the hydrogen sulfide-containing gas and/or inhalation of said gas as well as observation of a skin excision>

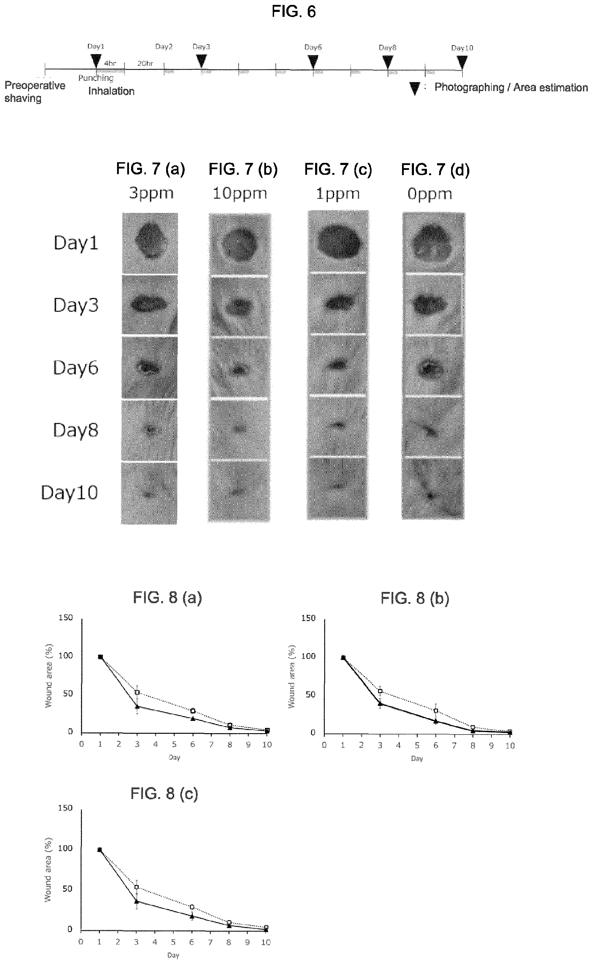

After the aforesaid mouse was placed in the container 10 of the wound-treating apparatus 1 constructed as shown in Fig. 1, a gas having a hydrogen sulfide concentration of 3 ppm with air as a carrier gas was fed, and the skin excision was observed. Specifically, just after the mouse of which the back skin was hollowed out was placed in the container 10, a cycle comprising 4-hours gas feeding and 20-hours interruption of gas feeding was repeated nine times wherein photographs of the skin excision were taken before the start of gas feeding (Day 1 (standing for the number of elapsed days from the preoperative shaving, as will apply hereafter), after the completion of 2 cycles (Day 3), after the completion of 5 cycles (Day 6), after the completion of 7 cycles (Day 8) and after the completion of 9 cycles (Day 10) under anesthesia by isoflurane,respectively, and submitted to a third person dermatologist to get him or her to make a morphological estimation of healing process by area measurement. The photographs taken of the skin excision are attached hereto as Fig. 7(a), and the results of skin excision area measurement are indicated by black triangular markers and a solid line in Fig. 8(a), respectively.

### [Examples 6 and 7]

The treatments according to Examples 6 and 7 were carried out as in Example 5 except that the concentration of hydrogen sulfide in the gas fed into the container 10 was set at 10 ppm (Ex. 6), and 1 ppm (Ex. 7), respectively.

Referring to Example 6, photographs taken of the skin excision are attached hereto as Fig. 7(b), and the results of skin excision area measurement are indicated by black triangular markers and a solid line in Fig. 8(b), respectively. Referring to Example 7, photographs taken of the skin excision are attached hereto as Fig. 7(c), and the results of skin excision area measurement are indicated by black triangular markers and a solid line in Fig. 8(c), respectively.

### [Comparative Example 3]

The treatment according to Comparative Example 3 was carried out as in Example 5 except that the gas fed into the container 10 was only an air, or a gas having a hydrogen sulfide concentration of 0 ppm. Photographs taken of the skin excision were attached hereto as Fig. 7(d), and the results of skin excision area measurement are indicated by white square markers and a dotted line in Fig. 8(a) to Fig. 8(c), respectively.

From comparisons of Figs. 7(a) to 7(c) with Fig. 7(d), it has been identified that the skin excision state is slightly improved from the completion of two cycles (Day 3) to the completion of five cycles (Day 6) by exposure to the hydrogen sulfide-containing gas and/or inhalation of said gas. Referring to Fig. 8, it has been identified that in the mouse exposed to the hydrogen sulfide-containing gas and/or allowed to inhale said gas, the skin excision area decreases noticeably from the completion of two cycles (Day 3) to the completion of five cycles (Day 6).

The foregoing results would imply that the exposure to a gas containing hydrogen sulfide in a proportion of 20 ppm or lower and inhalation of said gas have a healing efficacy against not only bedsores but also general wounds.

### [Example 8]

In Example 8 as well as Comparative Example 4 to be given later, a diabetic mouse was used as the living organism to be treated to make a study of the inventive effect. As a diabetic living organism decreases in terms of wound natural healing power, some noticeable wound healing effect would be expected by exposure to the hydrogen sulfide gas and/or allowing the living organism to inhale said gas.

The treatment according to Example 6 was carried out as in Example 6 except that a diabetic mouse (C57BL/6J HamSlc-ob/ob mouse at 8-weeks old) was used as the living organism to be treated. Note here that the diabetic mouse used is an obese one as a model of type II diabetes.

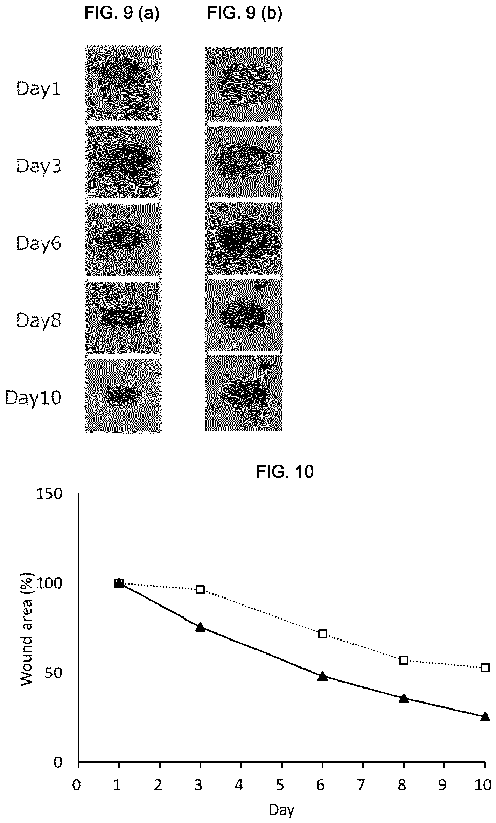

Photographs taken of the skin excision are attached hereto as Fig. 9(a), and the results of skin excision area measurement are indicated by black triangular markers and a solid line in Fig. 10, respectively.

### [Comparative Example 4]

The treatment according to Comparative Example 4 was carried out as in Comparative Example 3 except that the aforesaid diabetic mouse was used.

Photographs taken of the skin excision are attached hereto as Fig. 9(b), and the results of skin excision area measurement are indicated by white square markers and a dotted line in Fig. 10, respectively.

From comparisons of Fig. 9(a) with Fig. 9(b), it has been identified that the skin excision state is noticeably improved by exposure to the hydrogen sulfide-containing gas and/or allowing the living organism to inhale said gas from after the completion of two cycles (Day 3). Referring to Fig. 10, it has been identified that in the inventive example comprising exposure to the hydrogen sulfide-containing gas and/or inhalation of said gas rather than the comparative example without the exposure and/or the inhalation, the area of the skin excision decreases noticeably after the completion of two cycles (Day 3).

The foregoing results would reveal that the exposure to a gas containing hydrogen sulfide in a proportion of 20 ppm or lower and/or inhalation of said gas are especially efficacious against living organisms whose natural healing power remains low due to diseases such as diabetes.

### Industrial Applicability

According to the present invention, it is possible to provide a novel method for treating wounds. The present invention is thus of great value in that the number of wound-treating options increase, and proper treatment is feasible depending on patient's conditions and constitutions as well as demands for treating methods.

### Explanation of the Reference Numerals

1: Wound-treating apparatus
10: Cover or container
20: Gas source for wound treatment
30: Carrier gas source

## Claims

1. A wound-treating gas composition, which contains hydrogen sulfide in a proportion of 20 ppm or lower.

2. A wound-treating gas composition, which contains hydrogen sulfide in a proportion of 10 ppm or lower.

3. The gas composition according to claim 1 or 2, wherein the wound-treating gas composition is provided to treat a bedsore.

4. A wound-treating apparatus, comprising a cover or container configured to receive the gas composition according to any one of claims 1 to 3 and expose a wound to said gas composition and/or allow a living organism to be treated to inhale said gas.

5. The wound-treating apparatus according to claim 4, wherein the container is configured to be closable while taking in the whole body of the living organism to be treated.

6. The wound-treating apparatus according to claim 4 or 5, which is configured such that delivery and interruption of said gas composition are intermittently carried out within a preset time.
